# EUROPÄISCHE PATENTSCHRIFT

(11) **EP 0 678 515 B1**
(45) Veröffentlichungstag und Bekanntmachung des Hinweises auf die Patenterteilung: **08.07.1998**
(21) Anmeldenummer: 95105830.4
(22) Anmeldetag: 19.04.1995
(51) Int. Cl.: C07D 307/33, C07C 255/12

(54) **Verfahren und Zwischenprodukte zur Herstellung von 5-Oxaspiro [2.4] heptan-6-on**
Process and intermediates for the production of 5-oxaspiro [2.4] heptan-6-one
Procédé et intermédiaires pour la production de 5-oxaspiro [2.4] heptan-6-one

(30) Priorität: 21.04.1994 CH 1230/94
(43) Veröffentlichungstag der Anmeldung: 25.10.1995
(73) Patentinhaber: LONZA AG, CH-3945 Gampel/Wallis (CH)
(72) Erfinder: Hanselmann, Paul, Dr., CH-3930 Visp (Kanton Wallis) (CH)
(74) Vertreter: Weinhold, Peter, Dr.

(56) Entgegenhaltungen:
- EP-A- 0 480 717

## Beschreibung

Die vorliegende Erfindung betrifft ein neues Verfahren zur Herstellung von 5-Oxaspiro[2.4]heptan-6-on der Formel 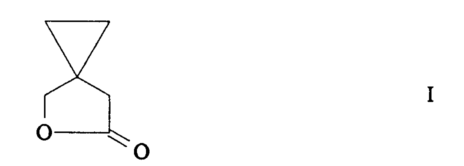 sowie neue Zwischenprodukte des erfindungsgemässen Verfahrens.

5-Oxaspiro[2.4]heptan-6-on (I) ist ein Zwischenprodukt zur Herstellung von Leukotrien-Antagonisten (EP-A 0 480 717). Eine bekannte Synthese dieser Verbindung geht vom Itaconsäureester aus und endet mit der Reduktion des cyclischen Anhydrids 5-Oxaspiro[2.4]heptan-4,6-dion zur Titelverbindung. Diese Reduktion liefert jedoch ein Gemisch des gewünschten Produkts mit dem isomeren 5-Oxaspiro[2.4]heptan-4-on und ist deshalb für die preiswerte Herstellung grosser Mengen wenig geeignet.

Aufgabe der vorliegenden Erfindung war daher, ein Verfahren zur Herstellung von 5-Oxaspiro[2.4]heptan-6-on bereitzustellen, welches von gut zugänglichen Materialien ausgeht und keine Isomerentrennung erfordert.
Erfindungsgemäss wird die Aufgabe durch das Verfahren nach Patentanspruch 1 und die Zwischenprodukte nach Patentansprüchen 5 bis 7 gelöst.
Es wurde gefunden, dass das [3-(Brommethyl)oxetan-3-yl]acetonitril der Formel 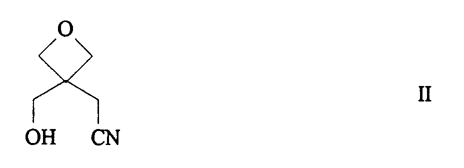 welches aus Pentaerythritdibromid leicht zugänglich ist (F. Govaert, P. Cornand, *Meded. K. Vlaam. Acad. Wet., Lett. Schone Kunsten Belg., Kl. Wet., **1954,** 16,* 1 ― 13), auf einfache Weise durch Behandeln mit Bromwasserstoff in das 4,4-Bis(brommethyl)dihydro-2-furanon der Formel 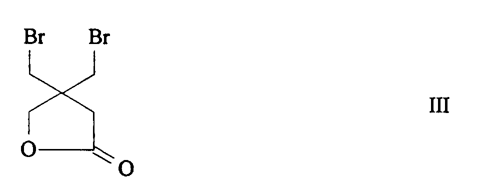 übergeführt werden kann. Die Bis(brommethyl)-Verbindung III kann anschliessend durch Behandeln mit Zink in guter Ausbeute zur Titelverbindung cyclisiert werden.

Die Überführung des Nitrils II in die Bis(brommethyl)-Verbindung III läuft je nach Reaktionsbedingungen unterschiedlich ab, so dass entweder isolierbare Zwischenprodukte entstehen, die in einer oder zwei zusätzlichen Stufen durch weitere Einwirkung von Bromwasserstoff in die Bis(brommethyl)-Verbindung III zu überführen sind, oder in einer einzigen Stufe direkt die Bis(brommethyl)-Verbindung III erhalten wird. Hierbei bezieht sich die Formulierung "in einer einzigen Stufe" nur auf die praktische Durchführung der Synthese und bedeutet nicht, dass die Reaktion tatsächlich in einem Schritt abläuft.
Bei Einsatz von wenig Bromwasserstoff, beispielsweise ca. 1 mol HBr auf 1 mol Nitril II, und relativ milden Bedingungen (Raumtemperatur) wird als erstes isolierbares Produkt das 3,3-Bis(hydroxymethyl)-4-brombutyronitril der Formel 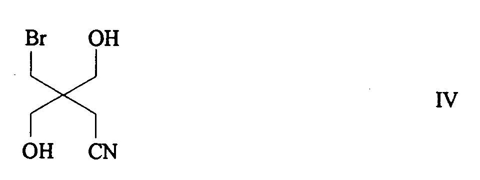 erhalten. Bei etwas schärferen Reaktionsbedingungen, beispielsweise 80°C und ca. 2 mol HBr auf 1 mol Nitril II, entsteht als isolierbares Produkt das 4-(Brommethyl)-4-(hydroxymethyl)dihydro-2-furanon der Formel 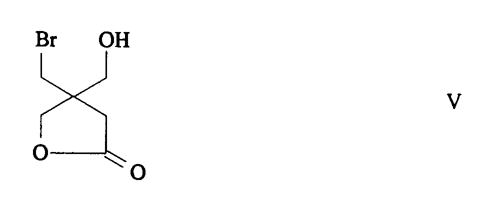 Sowohl das Bis(hydroxymethyl)nitril IV als auch das Brommethyl-hydroxymethyl-lacton V können durch weitere Umsetzung mit Bromwasserstoff in die Bis(brommethyl)-Verbindung III übergeführt werden. Hierfür sind allerdings drastischere Reaktionsbedingungen (beispielsweise 120 °C, 10 Äquivalente HBr) erforderlich.
Wird das Nitril II mit einem Überschuss an Bromwasserstoffbehandelt, d. h. mit mehr als 3 mol HBr auf 1 mol Nitril, dann gelingt die Überführung in die Bis(brommethyl)-Verbindung III in einer Stufe.

Als Lösungsmittel, in denen die Umsetzung des Nitrils II oder die weitere Umsetzung des Bis(hydroxymethyl)nitrils IV bzw. des Brommethyl-hydroxymethyl-lactons V mit Bromwasserstoff durchgeführt werden kann, eignen sich beispielsweise polare aprotische Lösungsmittel wie N,N-Dimethylformamid oder N,N-Dimethylacetamid, protische Lösungsmittel wie niedrige Carbonsäuren oder Wasser, sowie Mischungen dieser Lösungsmittel.
Besonders bevorzugt sind Essigsäure, polare aprotische Lösungsmittel allein oder im Gemisch mit Wasser oder Wasser allein.

Der Bromwasserstoff kann sowohl als handelsübliche Lösung in Wasser oder Essigsäure als auch gasförmig zugegeben bzw. in das Reaktionsgemisch eingeleitet werden.

Die Cyclisierung der Bis(brommethyl)-Verbindung III zum 5-Oxaspiro[2.4]heptan-6-on (I) mit Zink nach Art einer intramolekularen Wurtz-Synthese kann analog zu bekannten Synthesen anderer Verbindungen mit Cyclopropanring aus den entsprechenden 1,3-Dihalogen-Verbindungen erfolgen. Eine Übersicht über diesen Reaktionstyp findet sich beispielsweise bei D. Wendisch, *Methoden Org. Chem. (Houben-Weyl) 4. Aufl.* Bd. IV/3, S. 32 - 42. Als Lösungsmittel eigenen sich im vorliegenden Fall insbesondere niedrige Alkohole wie beispielsweise Ethanol oder Amide wie beispielsweise N,N-Dimethylacetamid.

Bei der Aufarbeitung ist zweckmässig darauf zu achten, dass stark basische bzw. nucleophile Bedingungen vermieden werden, da sonst der Lactonring geöffnet werden kann. Dies ist insbesondere in Gegenwart von Alkoholen möglich, wobei die entsprechenden Hydroxyester gebildet werden.

Die folgenden Beispiele verdeutlichen die Durchführung des erfindungsgemässen Verfahrens.

### Beispiele

### Beispiel 1

### [3-(Brommethyl)oxetan-3-yl]methanol

Eine Lösung von 25 g (94 mmol) 2,2-Bis(brommethyl)-1,3-propandiol (Pentaerythritdibromid) in 250 ml Ethanol wurde mit 6,1 g (94 mmol) Kaliumhydroxid (86%, Plätzchen) 2 h bei Raumtemperatur gerührt. Anschliessend wurde das entstandene Kaliumbromid abfiltriert und das Filtrat im Vakuum eingedampft. Das so erhaltene gelbliche Öl war ohne weitere Reinigung für die folgende Stufe verwendbar.
- Ausbeute (Rohprodukt):: 18,1 g (100%)
- Sdp.:: 86 °C / 0,2 Torr
- ¹H-NMR (CDCl₃, 300 MHz):: δ 4,49, 4,47 (AB-System, *J*_{AB} = 7,5 Hz, 4 H, Ring-H);
3,96 (s, 2 H, C*H*₂OH);
3,78 (s, 2 H, CH₂Br);
3,50 (s, 1 H, OH).

### Beispiel 2

### [3-(Hydroxymethyl)oxetan-3-yl]acetonitril (II)

Eine Lösung von 42 g (0,23 mol) [3-(Brommethyl)oxetan-3-yl]methanol (hergestellt nach Beispiel 1) in 420 ml Ethanol wurde mit 13 g (0,265 mol) Natriumcyanid 18 h am Rückfluss gekocht.
Nach dem Abkühlen auf Raumtemperatur wurde das ausgefallene Natriumbromid abfiltriert und das Filtrat eingeengt.
Der Rückstand wurde bei 0,4 Torr destilliert.
- Ausbeute:: 16 g (54%) farbloses Öl
- Sdp.:: 107 °C / 0,4 Torr
- ¹H-NMR (CDCl₃, 300 MHz):: δ 4,43 (s, 4 H, Ring-H);
3,97 (s, 2 H, C*H*₂OH);
2,85 (s, 2 H, CH₂CN);
2,50 (s, 1 H, OH).
- IR (Film):: ν=2247 cm⁻¹ (C≡N)

### Beispiel 3

### 3,3-Bis(hydroxymethyl)-4-brombutyronitril (IV)

Ein Gemisch aus 176 mg (1,39 mmol) [3-(Hydroxymethyl)oxetan-3-yl]acetonitril (II, hergestellt nach Beispiel 2) und 2,5 ml N,N-Dimethylacetamid wurde bei 10 °C mit 289 mg wässriger Bromwasserstoffsäure (48% HBr) versetzt. Nach dem Abklingen der exothermen Reaktion wurde das Gemisch auf Eis gegossen und anschliessend mit Diethylether extrahiert. Durch Einengen der Etherphase wurde ein Rohprodukt erhalten, das noch etwas N,N-Dimethylacetamid enthielt.
- Ausbeute:: 440 mg Rohprodukt, nach ¹H-NMR-Integration 64% der Theorie
- ¹H-NMR (CDCl₃, 300 MHz):: δ 3,95 (s, 2 H, OH);
3,67 (s, 4 H, C*H*₂OH);
3,54 (s, 2 H, CH₂Br);
2,59 (s, 2 H, CH₂CN).
- IR (Film):: ν = 2244 cm⁻¹ (C≡N)

### Beispiel 4

### 4-(Brommethyl)-4-(hydroxymethyl)dihydro-2-furanon (V)

Ein Gemisch aus 1,76 g (13,9 mmol) [3-(Hydroxymethyl)oxetan-3-yl]acetonitril (II, hergestellt nach Beispiel 2) und 5 ml N,N-Dimethylacetamid wurde bei Raumtemperatur mit 4,85 g wässriger Bromwasserstoffsäure (48% HBr) versetzt und nach dem Abklingen der exothermen Reaktion noch 2 h auf 90 °C erwärmt.
Anschliessend wurde das Reaktionsgemisch auf Eis gegossen und mit Ethylacetat extrahiert. Durch Einengen der organischen Phase wurde ein Rohprodukt erhalten, das noch etwas N,N-Dimethylacetamid enthielt.
- Ausbeute:: 1,45 g Rohprodukt, nach ¹H-NMR-Integration 39% der Theorie. ¹H-NMR (CDCl₃, 400 MHz): δ 4,28, 4,21 (AB-System, *J*_{AB} = 10 Hz, 2 H, Ring-CH₂O)
3,77 (s, 2 H, C*H*₂OH);
3,58 (s, 2 H, CH₂Br);
2,68 (s, 1 H, OH);
2,63, 2,56 (AB-System, *J*_{AB} = 16 Hz, 2 H, CH₂C=O).
- IR (Film):: ν = 1776 cm⁻¹ (C=O)

### Beispiel 5

### 4,4-Bis(brommethyl)dihydro-2-furanon (III)

Ein Gemisch aus 29 g (138 mmol) 4-(Brommethyl)-4-(hydroxymethyl)-dihydro-2-furanon (V, hergestellt nach Beispiel 4) und 69 g wässriger Bromwasserstoffsäure (48% HBr) wurde 5 h auf 120 °C erwärmt. Anschliessend wurde das Reaktionsgemisch auf Eis gegossen und mit Diethylether extrahiert. Beim Eindampfen des Ethers kristallisierte das Produkt aus.
- Ausbeute:: 23,7 g (63%)
- Schmp.:: 72 °C (H₂O)
- ¹H-NMR (CDCl₃, 300 MHz):: δ 4,28 (s, 2 H, CH₂O);
3,68 (s, 4 H, CH₂Br);
2,72 (s, 2 H, CH₂C=O).
- IR (KBr):: ν = 1786 cm⁻¹ (C=O)

### Beispiel 6

### 4,4-Bis(brommethyl)dihydro-2-furanon (III, einstufige Variante)

Bei Raumtemperatur wurden 1,0 g (7,3 mmol) [3-(Hydroxymethyl)oxetan-3-yl]acetonitril (II, hergestellt nach Beispiel 2 mit 8,93 g (36,6 mmol) Bromwasserstoff (30 proz. Lösung in Essigsäure) versetzt. Nach Abklingen der stark exothermen Reaktion wurde das Gemisch noch 1 h auf 100°C erwärmt.
Nach Abkühlen auf Raumtemperatur wurde das ausgefallene Salz (Ammoniumbromid) abfiltriert und das Filtrat bei 50 °C / 10 mbar zur Trockene eingedampft.
Der Rückstand wurde in Wasser / Diethylether aufgenommen und mit gesättigter Natriumhydrogencarbonatlösung neutralisiert. Nach Abtrennung der organischen Phase wurde die wässrige Phase noch dreimal mit Diethylether extrahiert.
Die vereinigten organischen Phasen wurden mit Natriumsulfat getrocknet und eingeengt. Es verblieben 1,85 g eines weissen Feststoffs mit einem Gehalt (nach GC) >90%.

### Beispiel 7

### 5-Oxaspiro[2.4]heptan-6-on (I)

In 2,5 ml N,N-Dimethylacetamid wurden bei Raumtemperatur 0,2 g (0,73 mmol) 4,4-Bis(brommethyl)dihydro-2-furanon (III, hergestellt nach Beispiel 5) und 80 mg (1,2 mmol) Zinkpulver vorgelegt und 5 h auf 120 °C erhitzt. Das Reaktionsgemisch wurde dann auf Eis gegossen und mit Ethylacetat extrahiert. Durch Eindampfen der organischen Phase wurden 80 mg Rohprodukt erhalten, das gemäss GC-MS noch etwas N,N-Dimethylacetamid enthielt.
- ¹H-NMR (CDCl₃, 300 MHz):: δ 4,19 (s, 2 H, CH₂O);
2,56 (s, 2 H, CH₂C=O);
0,70 - 0,78 (m, 4 H, Dreiring-H).
- IR (Film):: ν = 1777 cm⁻¹ (C=O)

### Beispiel 8

### 5-Oxaspiro[2.4]heptan-6-on (I)

In 20 ml N,N-Dimethylacetamid wurde bei Raumtemperatur 2,0 g (6,6 mmol) 4,4-Bis(brommethyl)dihydro-2-furanon (III, hergestellt nach Beispiel 5) und 0,89 g (13,2 mmol) Zinkpulver vorgelegt und 2 h auf 120 °C erwärmt. Nach Abkühlen auf Raumtemperatur wurden 2,4 g Ammoniakgas eingeleitet und der sich gebildete Niederschlag sowie das Zinkpulver (total 2,35 g) abfiltriert. Das Filtrat wurde bei 70 - 80 °C / 15 mbar eingedampft. Es verblieben 1,25 g einer gelblichen Flüssigkeit, welche nach GC aus 53% N,N-Dimethylacetamid und 46% Lacton bestand.
Zur endgültigen Reinigung wurde das Rohprodukt in Diethylether / Wasser aufgenommen und ergab nach Aufarbeitung und Kugelrohrdestillation das Spirolacton I (88 mg).

## Patentansprüche

1. Verfahren zur Herstellung von 5-Oxaspiro[2.4]heptan-6-on der Formel 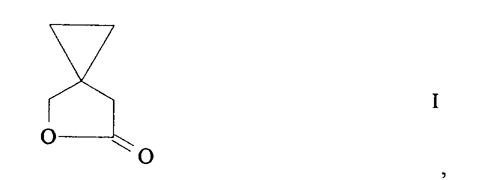 dadurch gekennzeichnet, dass [3-(Hydroxymethyl)oxetan-3-yl]acetonitril der Formel 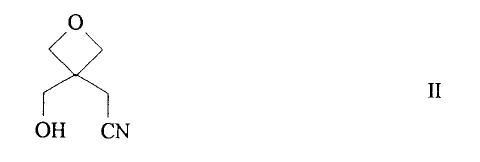 mit Bromwasserstoff in 4,4-Bis(brommethyl)dihydro-2-furanon der Formel 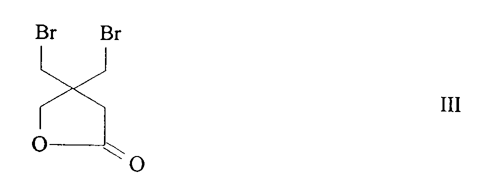 übergeführt und dieses mit Zink zur Titelverbindung cyclisiert wird.

2. Verfahren nach Anspruch 1, dadurch gekennzeichnet, dass die Überführung des Nitrils (II) in die Bis(brommethyl)-Verbindung (III) über das 3,3-Bis(hydroxymethyl)-4-brombutyronitril der Formel 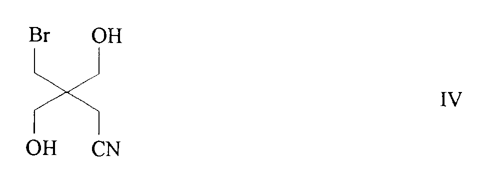 und / oder das 4-(Brommethyl)-4-(hydroxymethyl)dihydro-2-furanon der Formel 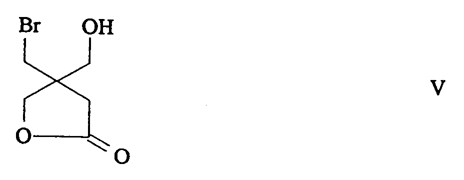 in zwei oder drei Stufen durchgeführt wird.

3. Verfahren nach Anspruch 1, dadurch gekennzeichnet, dass die Überführung des Nitrils (II) in die Bis(brommethyl)-Verbindung (III) in einer Stufe mit mehr als 3 mol HBr auf 1 mol Nitril durchgeführt wird.

4. Verfahren nach einem der Ansprüche 1 bis 3, dadurch gekennzeichnet, dass die Umsetzung mit Bromwasserstoff in Essigsäure oder einem polaren aprotischen Lösungsmittel und / oder Wasser durchgeführt wird.

5. 4,4-Bis(brommethyl)dihydro-2-furanon der Formel

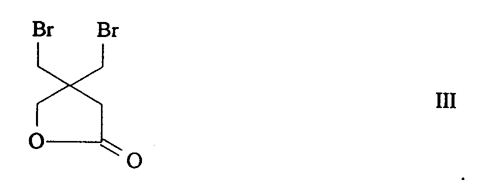

6. 3,3-Bis(hydroxymethyl)-4-brombutyronitril der Formel

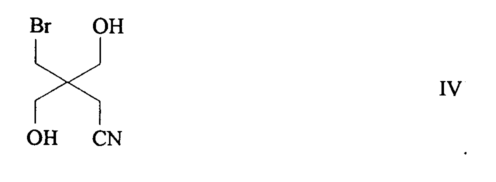

7. 4-(Brommethyl)-4-(hydroxymethyl)dihydro-2-furanon der Formel

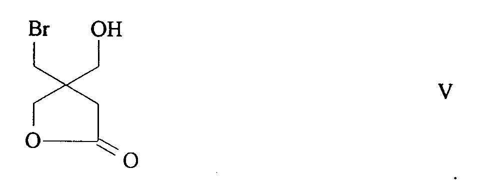

## Claims

1. A method for producing 5-oxaspiro[2.4]heptan-6-one having the formula 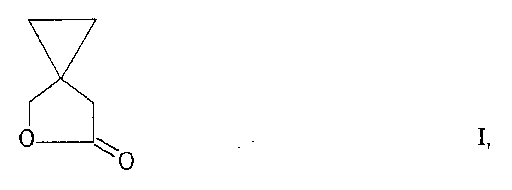 characterised in that [3-(hydroxymethyl)oxetan-3-yl]acetonitrile having the formula 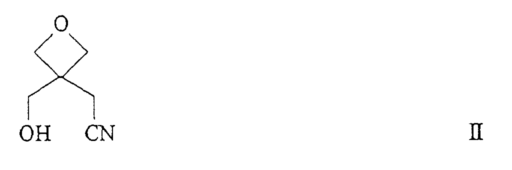 is reacted with hydrogen bromide to form 4,4-bis(bromomethyl)dihydro-2-furanone having the formula 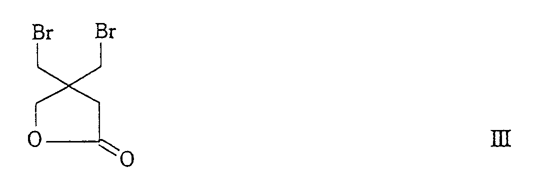 which is cyclised with zinc to form the compound specified in the title.

2. The method of claim 1, characterised in that transforming said nitrile (II) into said bis(bromomethyl) compound (III) is performed in two or three steps via the 3,3-bis(hydroxymethyl)-4-bromobutyronitrile having the formula 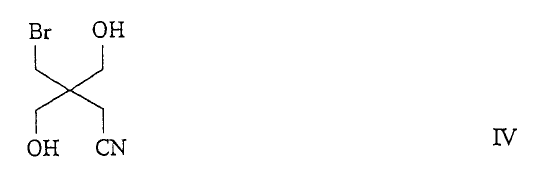 and/or the 4-(bromomethyl)-4-(hydroxymethyl)dihydro-2-furanone having the formula

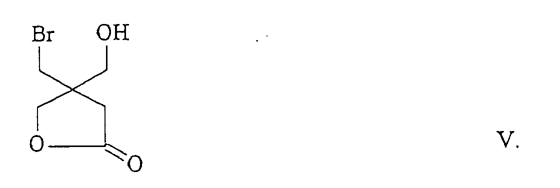

3. The method of claim 1, characterised in that transforming said nitrile (II) into said bis(bromomethyl) compound (III) is performed in one step with more than 3 mol HBr to 1 mol nitrile.

4. The method of one of claims 1 to 3, characterised in that the reaction is performed with hydrogen bromide in acetic acid or a polar aprotic solvent and/or water.

5. 4,4-bis(bromomethyl)dihydro-2-furanone having the formula

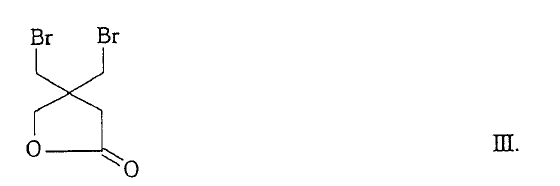

6. 3,3-bis(hydroxymethyl)-4-bromobutyronitrile having the formula

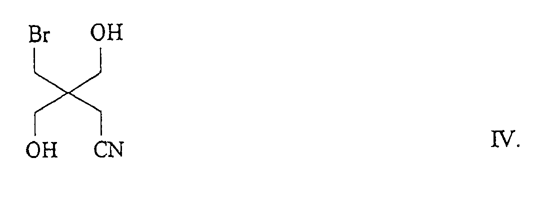

7. 4-(bromomethyl)-4-(hydroxymethyl)dihydro-2-furanone having the formula

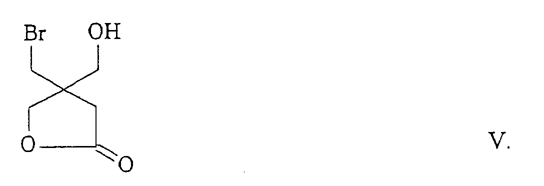

## Revendications

1. Procédé pour la préparation de 5-oxaspiro[2,4] heptan-6-one selon la formule 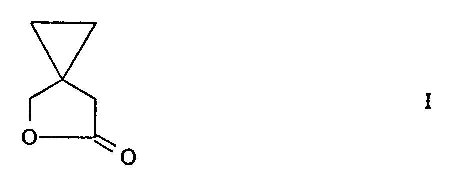 caractérisé en ce que le [3-(hydroxyméthyl)oxetan-3-yl]acétonitrile de la formule 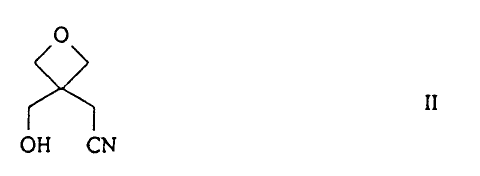 est transformé avec le bromure d'hydrogène en la 4,4-bis(bromométhyl)dihydro-2-furanone de la formule 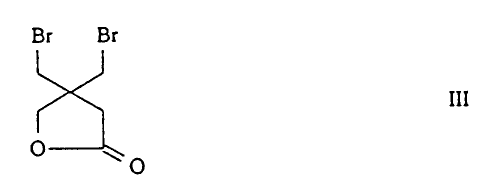 et celle-ci est cyclisée avec le zinc pour donner la composition du titre.

2. Procédé selon la revendication 1, caractérisé en ce que la transformation du nitrile (II) en le composé bis(bromométhyl) (III) est conduite en deux ou trois étapes par l'intermédiaire du 3,3-bis(hydroxyméthyl)-4-bromobutyronitrile de la formule 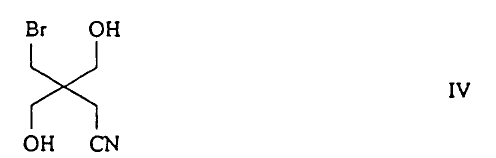 et/ou la 4-(bromométhyl)-4-(hydroxyméthyl)dihydro-2-furanone de la formule

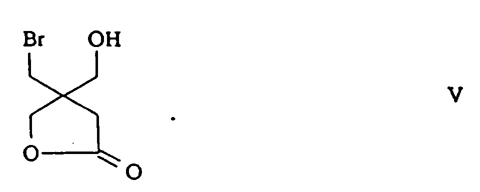

3. Procédé selon la revendication 1, caractérisé en ce que la transformation du nitrile (II) en le compose bis(bromométhyl) (III) s'effectue en une étape avec plus de 3 moles d'HBr pour 1 mole de nitrile.

4. Procédé selon l'une des revendications 1 à 3, caractérisé en ce que la réaction avec le bromure d'hydrogène est conduite dans l'acide acétique ou un solvant aprotique polaire et/ou de l'eau.

5. 4,4-bis(bromométhyl)dihydro-2-furanone de la formule

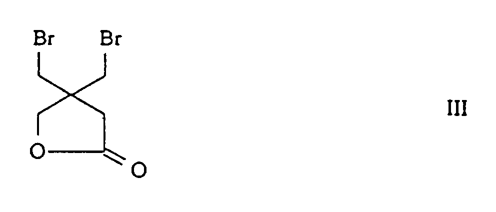

6. 3,3-bis(hydroxyméthyl)-4-bromobutyronitrile de la formule

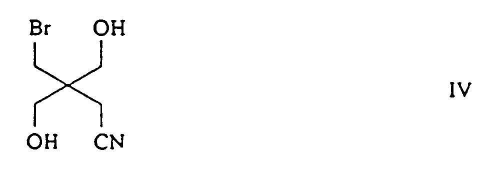

7. 4-(bromométhyl)-4-(hydroxyméthyl)dihydro-2-furanone de la formule
